(19) **Europäisches Patentamt**

**European Patent Office**

**Office européen des brevets**

(11) **EP 1 385 001 A1**

(12) **EUROPEAN PATENT APPLICATION**
published in accordance with Art. 158(3) EPC

(43) Date of publication:
**28.01.2004 Bulletin 2004/05**

(51) Int Cl.[7]: **G01N 33/543**, G01N 33/577,
G01N 33/573, G01N 33/545

(21) Application number: **02707280.0**

(22) Date of filing: **01.04.2002**

(86) International application number:
**PCT/JP2002/003255**

(87) International publication number:
**WO 2002/079782 (10.10.2002 Gazette 2002/41)**

(84) Designated Contracting States:
**AT BE CH CY DE DK ES FI FR GB GR IE IT LI LU MC NL PT SE TR**

(30) Priority: **30.03.2001 JP 2001099779**

(71) Applicant: **Mitsubishi Kagaku Iatron, Inc.**
**Tokyo 101-0031 (JP)**

(72) Inventors:
- **SAWAI, Tokio, Mitsubishi Kagaku Iatron, Inc**
  **Tokyo 101-0031 (JP)**
- **OHDE, Katsuya, Mitsubishi Kagaku Iatron, Inc**
  **Tokyo 101-0031 (JP)**

(74) Representative: **VOSSIUS & PARTNER**
**Siebertstrasse 4**
**81675 München (DE)**

(54) **REAGENT AND METHOD FOR IMMUNOANALYSIS OF ELASTASE 1 AND METHOD OF DETECTING PANCREATIC DISEASE**

(57)    A reagent for an immunological analysis of elastase 1, comprising two kinds of latex particles having different particle sizes wherein the latex particles carry two kinds of monoclonal antibodies having different specificities for elastase 1, is disclosed.

Further, a method for an immunological analysis of elastase 1, comprising the steps of: bringing a sample to be analyzed into contact with two kinds of latex particles having different particle sizes which carry two kinds of monoclonal antibodies having different specificities for elastase 1, and analyzing the degree of a latex-particles-agglutination caused by an antigen-antibody reaction, is disclosed.

Furthermore, a method for detecting a pancreatic disease wherein elastase 1 is analyzed by the above immunological analysis method is disclosed.

According to the present invention, elastase 1 can be conveniently and quickly (for example, for 10 minutes) analyzed, without a special equipment as in the case of RIA.

**EP 1 385 001 A1**

**Description**

TECHNICAL FIELD

The present invention relates to a reagent and a method for an immunological analysis of elastase 1 and a method of detecting a pancreatic disease. The term "analysis" as used herein includes a measurement to quantitatively or semi-quantitatively determine an amount of a substance to be assayed and a detection to judge a presence or absence of a substance to be assayed.

BACKGROUND ART

As pancreatic elastases, there are elastase 1 and elastase 2. Elastase 1 is secreted by the pancreas, and 90% thereof binds to $\alpha_1$-antitrypsin in blood. Measurement of an elastase concentration in blood is clinically useful: for this, elastase 1 is useful. Elastase is also located in leukocytes, platelets, and spleen, and granulocyte elastase, which relates to acute inflammation or the like, is distinguishable from the pancreatic elastases. The pancreatic elastases are exocrine pancreatic enzymes which specifically hydrolyze elastin, the fibrous protein of connective tissue, in blood. The pancreatic elastases are located as proelastase in acinar cells of pancreas, and secreted in the intestine and activated by trypsin. Elastase 1 reflects pancreatitis accompanied by a pancreatic cancer (particularly pancreatic head) and frequently shows an abnormal high value from an early stage, and thus is useful as a marker for the diagnosis of pancreatic diseases or useful in the follow-up thereof. Various methods for examining pancreatic diseases are known, and serum amylase has been measured in the past. However, amylase is also produced from organs other than the pancreas, and thus is not exactly specific as an examination of pancreatic diseases. Because elastase 1 is highly specific to pancreatic diseases, an accurate measurement can be obtained, which is very important in the medical field.
As a method for measuring elastase 1, a method for measuring an enzyme activity thereof was developed, but the sensitivity and accuracy thereof were not sufficient. An immunological method for measuring elastase 1 as an amount of protein is widely used, and RIA (radioimmunoassay) is widely used. More particularly, elastase 1 is analyzed by reacting elastase 1 in a sample to be analyzed and a radioisotope-labeled elastase 1 competitively against an elastase 1 antibody and measuring radioactivity which binds to the antibody, i.e., by a competitive method. However, in RIA, the measured values vary widely at a low value area, and the procedure is complicated and time-consuming. Further, various problems arise from using the radioisotope, such as the stability of a reagent (stability is lost after 60 days), a special measurement equipment in which the radioisotope can be handled, and a waste treatment. Further, facilities capable of performing RIA are limited, and thus it is difficult to carry out RIA in common medical facilities. An outsourcing of the examination becomes necessary, and thus the result cannot be obtained within one day. Therefore, although elastase 1 is an important item for diagnosing acute pancreatitis, it has a serious disadvantage in that the method cannot be used in an emergency examination. An EIA method using an enzyme instead of the radioisotope for labeling is proposed, but is also inappropriate for an emergency examination with respect to operativity and promptness. Further, a measurement accuracy at a low value area in the EIA method is poorer than that of the RIA method, and thus a situation does not arise in which the RIA method is actually replaced with the EIA method. In other words, using the RIA method to measure elastase 1 cannot be avoided, in spite of the above problems.
Elastase 1 shows a high value in a pancreatic cancer, but is a pancreas-specific enzyme, and thus is expected to become a marker for monitoring chronic pancreatitis, instead of amylase. For this purpose, it is important to improve conventional methods, in which a change in a low value area cannot be accurately measured.

DISCLOSURE OF INVENTION

The object of the present invention is to provide a reagent and a method for an immunological analysis of elastase 1, which can be analyzed conveniently without a special equipment, quickly so that an emergency examination can be carried out, and quantitatively in a wide range from a low concentration range to a high concentration range.
The present invention relates to a reagent for an immunological analysis of elastase 1, comprising two kinds of latex particles having different particle sizes wherein the latex particles carry two kinds of monoclonal antibodies having different specificities for elastase 1.
Further, the present invention relates to a method for an immunological analysis of elastase 1, comprising the steps of:

bringing a sample to be analyzed into contact with two kinds of latex particles having different particle sizes, the latex particles carrying two kinds of monoclonal antibodies having different specificities for elastase 1, and analyzing the degree of a latex-particles-agglutination caused by an antigen-antibody reaction.

Furthermore, the present invention relates to a method for detecting a pancreatic disease wherein elastase 1 is analyzed by the above method for an immunological analysis.

BRIEF DESCRIPTION OF DRAWINGS

Figure 1 is a graph showing a correlation between the method for an immunological analysis of the present invention and an RIA method.
Figure 2 is a graph showing a low detection limit in the method for an immunological analysis of the present invention.
Figure 3 is a graph showing a functional sensitivity in the method for an immunological analysis of the present invention.
Figure 4 is a graph showing a dilution parallelism in the method for an immunological analysis of the present invention, at a concentration range of 0 to 6000 ng/dL as a theoretical value.
Figure 5 is a graph showing a dilution parallelism in the method for an immunological analysis of the present invention, at a concentration range of 0 to 600 ng/dL as a theoretical value.

BEST MODE FOR CARRYING OUT THE INVENTION

The present invention will be explained in detail hereinafter.
The reagent for an immunological analysis of the present invention comprises at least two kinds of latex particles having different particle sizes, wherein the latex particles carry two kinds of monoclonal antibodies having different specificities for elastase 1, preferably a combination of

(1) the first latex particles carrying the first monoclonal antibody specific to elastase 1, and
(2) the second latex particles having a particle size different from that of the first latex particle and carrying the second monoclonal antibody having a specificity different from that of the first monoclonal antibody.

The latex particle used in the present invention is not particularly limited, so long as it can be used in a conventional reagent for an immunological analysis. As the latex particles, there may be mentioned, for example, latex particles of polystyrene or styrene-styrene sulfate copolymer.
An average particle size of the latex particles may be appropriately selected in accordance with a measuring equipment or a concentration of a sample to be analyzed. The average particle size may be, for example, 0.05 to 0.5 $\mu$m. Two kinds of latex particles having different particle sizes are used in the present invention, and thus an accurate analysis can be carried out in a wide range of from a low concentration to a high concentration. The term "average particle size of the latex particles" as used herein means a value measured by an electron microscope.
As smaller latex particles, for example, latex particles having a particle size of preferably 0.05 to 0.3 $\mu$m, more preferably 0.05 to 0.25 $\mu$m may be used. As larger latex particles, for example, latex particles having a particle size of preferably 0.2 to 0.5 $\mu$m, more preferably 0.25 to 0.5 $\mu$m may be used. A combination of latex particles having the above particle sizes is used in the present invention, and thus a detectable limit at the side of a low concentration can be improved and a good reproducibility can be obtained. At the side of a high concentration, misunderstanding caused by a decrease of an agglutination property (i.e., prozone phenomenon in which an agglutination property is decreased due to an excess high concentration, agglutination is seemingly decreased, and a measured value lower than an actual value is obtained) can be avoided, even if a concentration of elastase 1 is high.
As the anti-elastase 1 antibody carried on the latex, two kinds of monoclonal antibodies having different specificities, i.e., two kinds of monoclonal antibodies which recognize different epitopes, are used in the present invention. The monoclonal antibodies are not particularly limited, so long as they are anti-elastase 1 monoclonal antibodies in which the sites specifically recognized by each antibody are different. Elastase 1 in blood generally forms a complex with $\alpha_1$-antitrypsin. Therefore, it is preferable to use anti-elastase 1 monoclonal antibodies which specifically bind to the complex of elastase 1 and $\alpha_1$-antitrypsin at sites different to each other, and can cause only an agglutination of the complex of elastase 1 and $\alpha_1$-antitrypsin when used as latex particles carrying the anti-elastase 1 monoclonal antibodies. It is more preferable that the anti-elastase 1 monoclonal antibodies do not agglutinate free elastase 1.
In addition, it is necessary that one monoclonal antibody (hereinafter sometimes referred to as the first monoclonal antibody in the present invention) in the present invention, and another monoclonal antibody (hereinafter sometimes referred to as the second monoclonal antibody in the present invention) used in the combination with the first monoclonal antibody recognize different epitopes. Further, it is necessary that the distance between the epitopes is a distance in which a steric hindrance does not occur when used as a latex reagent.

In the present invention, two kinds of monoclonal antibodies and two kinds of latex particles having different particle sizes are used in an appropriate combination thereof. For example, the first monoclonal antibody can be carried on one of two kinds of latex particles (hereinafter sometimes referred to as the first latex particles in the present invention), and the second monoclonal antibody can be carried on another kind of latex particles (hereinafter sometimes referred to as the first latex particles in the present invention). Alternatively, two kinds of monoclonal antibodies (i.e., the first and second monoclonal antibodies) can be carried on the first latex particles, and two kinds of monoclonal antibodies can be carried on the second latex particles. The optimum conditions in the above structures can be appropriately selected in accordance with, for example, the sizes and/or ratio of the two kinds of latex particles, and/or the properties (such as a reaction rate) of monoclonal antibodies.

Further, a mixing ratio of latex particles having a larger particle size and those having a smaller particle size is not particular limited, but may be selected within, for example, 10:1 to 1:10.

The latex particles carrying the monoclonal antibodies can be prepared in accordance with a known method, such as sensitization by a physical or chemical bond. The monoclonal antibody may be an immunoglobulin molecule, or a fragment thereof, such as Fab' or $F(ab')_2$. $F(ab')_2$ is a preferred antibody. When IgG (i.e., not an antibody fragment) is used as the monoclonal antibody, non specific binding, in which a value very much higher than an actual amount of elastase 1 is observed, may sometimes occur in some samples derived from patients, whereas the non specific binding can be suppressed when $F(ab')_2$ is used.

The reagent for an immunological analysis of the present invention may contain various additives which may be added to a latex reagent, for example, a buffer, an agent for promoting agglutination (for example, a water-soluble polymer such as polyethylene glycol), an agent for suppressing a non-specific reaction (for example, an alkali metal salt or saccharides), or a protein [for example, bovine serum albumin (BSA)], in addition to the latex particles carrying the monoclonal antibodies.

As the above buffer, a buffer having a buffer capacity at pH 6 to 8.5 is preferable. The buffer of pH 6 to 8.5 is a well-known buffer and may be, for example, a tris buffer, a phosphate buffer, or a Good's buffer.

For example, when a tris buffer is used in the reagent for an immunological analysis of the present invention, a concentration of tris in the tris buffer is not particularly limited, so long as a desired tris concentration described below can be obtained in a system in which a latex agglutination reaction is carried out. The preferred concentration of the tris buffer is 0.1 to 0.5 mol/L.

A concentration of tris in a system in which a latex agglutination reaction is carried out is not particularly limited, so long as a self-agglutination of latex particles can be suppressed. The tris concentration can be appropriately selected in accordance with concentrations of additives (such as a salt, a protein, and/or saccharides) which coexist in the reaction system. The tris concentration in the system in which a latex agglutination reaction is carried out is preferably 0.1 to 0.5 mol/L, more preferably 0.2 to 0.3 mol/L. When the tris concentration is less than 0.1 mol/L, a self-agglutination of latex particles sometimes occurs. When the tris concentration is more than 0.5 mol/L, an antigen-antibody reaction is sometimes suppressed, and thus a low detection limit becomes worse.

A pH value of the buffer is preferably 6 to 8.5. When the pH value is outside of this range, a self-agglutination of latex particles or disadvantage in a measurement accuracy sometimes occurs.

When the reagent for an immunological analysis of the present invention contains a buffer of pH6 to 8.5, the buffer of pH6 to 8.5 and the latex particles carrying the monoclonal antibodies can be contained in the reagent in any form, so long as the latex particles, the buffer, and a sample to be analyzed can be brought into contact with each other in a latex agglutination reaction when in use. In other words, the form of the reagent for an immunological analysis of the present invention is not particularly limited in the above case. For example, the reagent may be a one reagent-component system containing the buffer of pH6 to 8.5 and the latex particles carrying the monoclonal antibodies, or a two reagent-components system composed of a first reagent-component containing the latex particles carrying the monoclonal antibodies and a second reagent-component containing the buffer of pH6 to 8.5.

In the method for an immunological analysis of the present invention, elastase 1 in a sample to be analyzed can be analyzed by bringing the sample into contact with the latex particles carrying the antibodies, preferably under the condition of pH6 to 8.5, to thereby cause an antigen-antibody reaction and a latex agglutination reaction, and then analyzing the degree of agglutination.

The sample to be analyzed which can be analyzed by the method for an immunological analysis of the present invention is not particularly limited, so long as it is a sample which may contain elastase 1. The sample to be analyzed may be, for example, a sample from a living body, more particularly, the serum, plasma, urine, pancreatic fluid, or humor.

When a latex agglutination reaction is carried out under the condition of pH6 to 8.5, by using the buffer of pH6 to 8.5 in the method for an immunological analysis of the present invention, the latex particles carrying the monoclonal antibodies, the buffer of pH6 to 8.5, and a sample to be analyzed can be brought into contact with each other in any sequence, so long as an antigen-antibody reaction does not occur in the absence of the buffer of pH6 to 8.5 (i.e., the latex particles are not brought into contact with the sample at first). For example, the latex particles carrying the monoclonal antibodies can be brought into contact with the buffer of pH6 to 8.5, and then the mixture can be brought into contact with the sample to be analyzed. Alternatively, the sample to be analyzed can be brought into contact with the buffer of pH6 to 8.5, and then the mixture can be brought into contact with the latex particles carrying the monoclonal antibodies.

The conditions of an antigen-antibody reaction in the method for an immunological analysis of the present invention may be the same as those in an ordinary immunological latex turbidimetry method. For example, the reaction pH is preferably 6 to 8.5. The reaction temperature is preferably 0 to 50°C, more preferably 20 to 40°C. The reaction time may be appropriately selected. For example, the measurement using an automated biochemical analyzer can be finished within 10 to 15 minutes.

The degree of the agglutination caused by the antigen-antibody reaction can be analyzed by a known method such as an optical analysis method. As the optical analysis method, there may be mentioned, for example, a method for analyzing scattered light or transmitted light by radiating light to the reaction solution. More particularly, the optical analysis can be carried out using an optical equipment to measure the strength of scattered light, absorbance, or strength of the transmitted light. The preferred wave length to be measured is 300 to 800 nm. The analysis using the optical equipment can be carried out in accordance with a known method, more particularly, by selecting particle sizes and/or concentrations of the latex particles and the reaction time and measuring an increase or decrease in strength of scattered light, absorbance, or strength of the transmitted light. Further, the above methods can be used in combination thereof.

In the method for detecting a pancreatic disease of the present invention, a pancreatic disease (particularly acute pancreatitis) can be detected or diagnosed by using a serum or plasma as a sample to be analyzed and analyzing elastase 1 in the sample to be analyzed by the method for an immunological analysis of the present invention. More particularly, when an amount of elastase 1 in a serum or plasma is 400 ng/dL or more, the sample can be judged as that from a patient suffering from a pancreatic disease (particularly acute pancreatitis).

The measurement of elastase 1 has advantages in comparison with conventional markers for a pancreatic disease. For example, when suffering from a stomach ache but visiting a hospital after several days therefrom (i.e., not an emergency case), conventional markers such as amylase or lipase are often normalized. In contrast, a biological half-life of elastase 1 in blood is so long that, if elastase 1 shows an abnormal value when visiting, the abnormal value may be an evidence (a late marker) which shows a disorder of pancreas (or pancreatic duct) at least several days ago, and thus it becomes necessary to carry out a clinical examination, diagnosis, and treatment, including a possibility of a pancreatic cancer. In other words, a period from the alleviation of an acute symptom to normalization with respect to elastase 1 is longer (for example, a week to a month) than that (several days) of amylase or lipase. From this point, the present invention in which elastase 1 can be measured quickly, conveniently, and with sensitivity has an excellent availability.

For example, an accurate measurement at or near the above concentration (400ng/dL) is necessary to judge the existence of acute pancreatitis. Such a concentration cannot be accurately measured by an EIA method, and thus only an RIA method can be selected among conventional methods. However, a measurement accuracy (reproducibility) is not sufficient at the above concentration range even in the RIA method. The above problems can be solved by the present invention. Because of the use of a latex method, elastase 1 can be accurately measured in a wide range, and the present invention has a remarkable effect in that a pancreatic disease can be accurately detected.

According to the present invention, in which a low value of elastase can be accurately measured, chronic pancreatitis can be accurately monitored, and thus various pancreatic diseases can be widely judged. In the past, the monitoring of chronic pancreatitis has depended on amylase, which is not exactly specific to pancreas. According to the present invention, elastase 1 from low values to high values, which cannot be measured by a conventional method, can be measured conveniently and quickly, without an additional procedure such as a dilution of a sample or increase of an amount applied. The above effects have excellent advantages in the field of clinical examination.

As described above, the reagent for measuring elastase 1 by a latex agglutination reaction system according to the present invention is novel, and a pancreatic disease can be accurately detected by accurately measuring elastase 1 using the latex agglutination reagent.

EXAMPLES

The present invention now will be further illustrated by, but is by no means limited to, the following Examples.

Example 1: Preparation of the reagent for an immunological analysis of the present invention (two reagent-components system)

In this example, the reagent for an immunological analysis of the present invention, which is a two reagent-components system composed of a first reagent-component containing a buffer of pH 6 to 8.5 and a second reagent-component containing the first latex particles carrying the first monoclonal antibody and the second latex particles carrying the second monoclonal antibody, was prepared.

(1) Preparation of anti-elastase 1 monoclonal antibodies

Hybridomas producing an antibody which can recognize a complex of elastase 1 and $\alpha_1$-antitrypsin were selected in accordance with a conventional method, except that elastase 1 was used as an immunogen and that elastase 1 and the complex of elastase 1 and $\alpha_1$-antitrypsin were used as antigens for screening. Further, antibodies which can cause a latex agglutination reaction with only the complex of elastase 1 and $\alpha_1$-antitrypsin were selected from among antibodies produced by the resulting hybridomas, and then anti-elastase 1 antibody fragments F(ab')$_2$ for the immunological analysis reagent were prepared.

The detailed procedure is as follows:

An immunogen solution of elastase 1 (A280 nm = 1.0) was mixed with an equal volume of complete Freund's adjuvant until emulsified, and then, 0.1 mL of the mixture was administered intraperitoneally in mice to immunize the same (first immunization). After 30 days, the above mixture was administered intraperitoneally to the mice in the same manner (second immunization). After 21 days from the second immunization, 0.1 mL of a diluted solution prepared by diluting the immunogen solution of elastase 1 (A280 nm = 1.0) with an equal volume of a physiological saline was administered intravenously to the mice (final immunization). After 3 days from the final immunization, the spleens were removed aseptically from the mice. The spleen cells (1 x 10$^8$) were added to pre-cultivated mouse myeloma cells SP2/0-Ag14 (about 2 $\times$ 10$^7$) to carry out a cell fusion in the presence of 40% polyethylene glycol 4000. The cell suspension was poured into each well of 96-well cell cultivation plates in an amount of 0.1 mL, and cultivated in an HAT medium for two weeks. After the cultivation, the presence of antibodies in the culture supernatants was confirmed. Hybridomas producing an antibody were cloned by a limiting dilution method, to obtain 8 clones producing an antibody which reacts with the complex of elastase 1 and $\alpha_1$-antitrypsin. In this connection, the complex was prepared by mixing an elastase 1 solution and an $\alpha_1$-antitrypsin solution at 37°C for 3 hours and being allowed to stand at 4°C overnight.

Bristane (2,6,10,14-tetramethylpentadecane, 0.5 mL) was administered intraperitoneally into 10 to 12 week old BALB/C mice. After 14 to 20 days, each hybridoma (1 $\times$ 10$^7$) was inoculated into the abdominal cavities of the mice and proliferated in vitro to obtain each anti-elastase 1 antibody. Each monoclonal antibody was carried on polystyrene latex particles, and two kinds of anti-elastase 1 antibodies E1 and E4 were selected, as the antibody which can cause an agglutination reaction with only the complex of elastase 1 and $\alpha_1$-antitrypsin. To each monoclonal antibody, 2.5% of pepsin was added. The digestion was carried out in an incubator at 37°C for 16 hours, and stopped by increasing the pH to 8 with a tris solution. Each reaction mixture was applied to a gel filtration chromatography using a Sephacryl S-200 column to pool each antibody fragment F(ab')$_2$ fraction.

In this connection, the complex of elastase 1 and $\alpha_1$-antitrypsin can be used as an immunogen.

(2) Preparation of a solution containing latex sensitized with anti-elastase 1 monoclonal antibodies

To 9 mL of a solution prepared by dissolving the F(ab')$_2$ fraction of the anti-elastase 1 antibody E1 [prepared in Example 1(1)] at a concentration of 0.2 mg/mL in a 0.01 mol/L tris buffer (pH 8.0), 1 mL of a polystyrene latex solution (average particle size = 0.2 $\mu$m, solid content = 10% by weight) was added, and the mixture was stirred at room temperature for 60 minutes. A tris buffer (pH 8.0) containing 0.5% by weight of bovine serum albumin (BSA) was added to the mixture. The whole was stirred at room temperature for 60 minutes, and centrifuged at 20000 rpm. The latex was suspended by adding 10 mL of a tris buffer (pH 8.0) to the resulting pellets, to prepare a solution containing latex sensitized with anti-elastase 1 antibody (E1).

The above procedure was repeated, except that the F(ab')$_2$ fraction of the anti-elastase 1 antibody E4 [prepared in Example 1(1)] was used as the antibody, and that polystyrene latex (average particle size = 0.3 $\mu$m, solid content = 10% by weight) was used as the polystyrene latex, to prepare a solution containing latex sensitized with anti-elastase 1 antibody (E4). Two latex solutions were mixed and diluted with a tris buffer so that an optical density (OD) at 700 nm became approximately 2, to prepare a solution containing latex sensitized with anti-elastase 1 monoclonal antibodies.

(3) Preparation of a tris buffer

BSA was dissolved at a concentration of 5.0% by volume in 0.2 mol/L of a tris buffer, and then adjusted to pH 8.2.

Comparative Example 1: Preparation of a reagent for an immunological analysis for comparison

The procedure described in Example 1(2) for preparing the solution containing latex sensitized with anti-elastase 1 antibody (E1) was repeated, except that the F(ab')$_2$ fraction of the anti-elastase 1 antibody E4 [prepared in Example 1(1)] was used as the antibody, to prepare a solution containing latex sensitized with anti-elastase 1 antibody (E4). The resulting latex solution (latex particle size = 0.2 μm) and the solution containing latex sensitized with anti-elastase 1 antibody (E1) [prepared in Example 1(2), latex particle size = 0.2 μm] were mixed and diluted with a tris buffer so that an optical density (OD) at 700 nm became approximately 2, to prepare a solution of latex having a single latex particle size and sensitized with anti-elastase 1 monoclonal antibodies.

As a tris buffer, the tris buffer prepared in Example 1(3) was used.

Evaluation

(1) Confirmation of the relationship between an RIA method and the method for an immunological analysis of the present invention

For 80 human serum and plasma samples in which an amount of elastase 1 contained therein had been previously determined by an RIA method, an amount of elastase 1 in each samples was determined using the reagent for an immunological analysis of the present invention prepared in Example 1, in accordance with the following procedure.

To 5 μL of each human serum and plasma, 195 μL of the tris buffer prepared in Example 1(3) was added, and the mixture was incubated at 37 °C. To the mixture, 65 μl of the solution containing latex sensitized with anti-elastase 1 monoclonal antibodies, prepared in Example 1(2), was added and stirred. After 5 minutes from the addition of the latex solution, absorbance at a wave length of 570 nm was measured. A change of absorbance during the period was regarded as an amount of change in absorbance (ΔAbs). The measurement was carried out using an automated analyzer (Hitachi 7170, Hitachi Ltd.).

In this connection, the RIA method was carried out as follows. To 100 μL of each human serum and plasma, 100 μL of an iodinated elastase 1 solution and 100 μL of a first antibody (anti-human elastase 1 rabbit antibody) were added. The mixture was incubated at 37 °C for 3 hours, and then 500 μL of a second antibody (anti-rabbit IgG goat antibody) specific to the first antibody was further added and mixed. The whole was centrifuged (1500 to 2200 G) at room temperature for 20 minutes. After removing the supernatant, radioactivity in the pellet was measured to determine an amount of elastase 1.

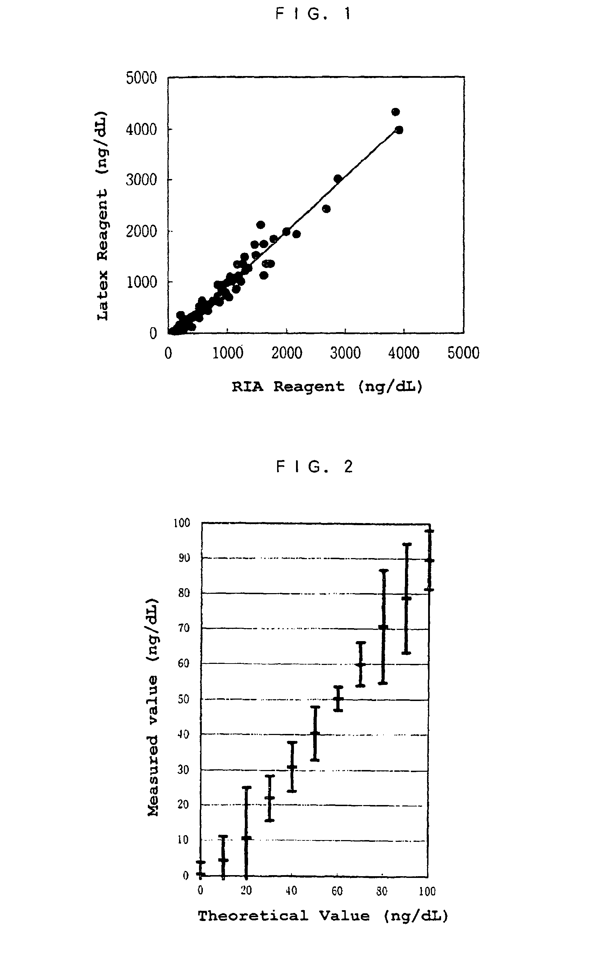

The result was shown in Fig. 1. Equation (1):

$$y=1.0639x-129.29 \tag{1}$$

was obtained as a correlation, and the R$^2$ value (R: coefficient of correlation) was 0.9674. The result showed a close correlation between the RIA method and the method for an immunological analysis of the present invention.

(2) Confirmation of low detection limit (functional sensitivity) in the method for an immunological analysis of the present invention

A human serum containing an elastase 1 antigen at a concentration of 100 ng/dL was used as a standard solution of an elastase 1 antigen. Diluted solutions were prepared by diluting the standard solution to 0 ng/dL, 10 ng/dL, 20 ng/dL, 30 ng/dL, 40 ng/dL, 50 ng/dL, 60 ng/dL, 70 ng/dL, 80 ng/dL, and 90ng/dL, and then a concentration of elastase 1 in each diluted solution was measured ten times per each diluted solution. The measurement was carried out in accordance with the procedure described in Evaluation (1), except that 10 μL of each diluted solution was used instead of 10 μL of the human serum.

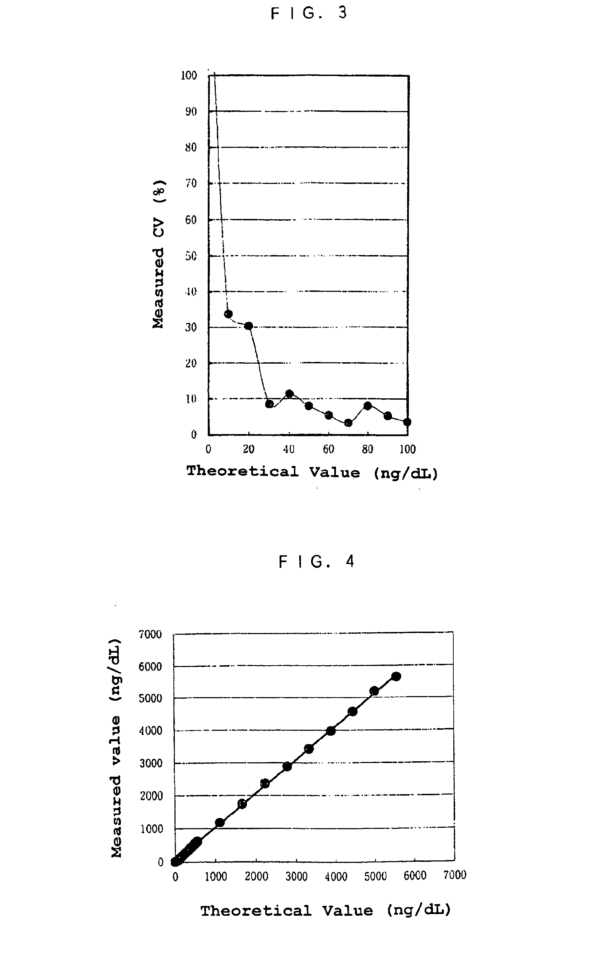

Each average (unit = ng/dL) of 10 measured values for an elastase 1 concentration in each diluted solution was as shown in Fig. 2, and each coefficient of variation (CV; unit = %) calculated from the measured values was as shown in Fig. 3. In Fig. 2, each value "average ± (2.6×SD)" (i.e., a value obtained by adding a value obtained by multiplying a standard deviation (SD) by 2.6 to the average, and a value obtained by subtracting the same from the average) was also shown. In Fig. 2, short horizontal lines located at the top, the middle, and the bottom of each vertical line means "average + (2.6×SD)", "average", and "average - (2.6×SD)", respectively.

In general, a minimum detectable sensitivity (low detection limit) can be determined from a minimum concentration in which the range "average ± (2.6×SD)" therein does not overlap with the range "average ± (2.6×SD)" in a control solution not containing elastase 1 (i.e., the value "average - (2.6×SD)" in the minimum concentration is higher than the value "average + (2.6×SD)" in the control solution). As shown in Fig. 2, the minimum detectable sensitivity (low detection limit) of the reagent for an immunological analysis of the present invention prepared in Example 1 was 30 ng/dL.

(3) Confirmation of precision (intra assay) in the method for an immunological analysis of the present invention

Each concentration of elastase 1 in three samples, in which an amount of elastase 1 is different from each other, was determined by the method for an immunological analysis of the present invention.

For comparison, each concentration of elastase 1 in the samples was determined by the reagent for an immunological analysis for comparison prepared in Comparative Example 1. Further, each concentration of elastase 1 in the samples was determined by an EIA method in accordance with the procedure described in Comparative Example 2.

The results in the three samples are shown in Tables 1 to 3, respectively. In Tables 1 to 3, "Example 1", "Comparative Example 1", and "Comparative Example 2" at the top row mean the results obtained by using the reagent for an immunological analysis of the present invention prepared in Example 1, those obtained by using the reagent for an immunological analysis for comparison prepared in Comparative Example 1, and those measured by the EIA method, respectively. In addition, Tables 1 to 3 show the results of the samples in which each concentration of elastase 1 was approximately 90 ng/dL, 900 ng/dL, and 2100 ng/dL, respectively.

As shown in Tables 1 to 3, reproducible measured values were obtained from low values to high values. In contrast, the latex agglutination method (Comparative Example 1) using one kind of latex particles having a same particle size, or the EIA method (Comparative Example 2) showed a poor reproducibility overall, and correct values were not obtained, particularly at low values. As above, it was found that when a combination of latex particles having different particle sizes (in this case, a combination of latex particles having small particle sizes) is not used, a quantitative measurement is not correctly carried out in a broad range.

Table 1

|  | Example 1 | Comparative Example 1 | Comparative Example 2 |
|---|---|---|---|
| Measured value (ng/dL) | 90 | 163 | 131 |
|  | 94 | 115 | 158 |
|  | 93 | 99 | 122 |
|  | 89 | 83 | 90 |
|  | 92 | 103 | 108 |
|  | 91 | 98 | 134 |
|  | 92 | 140 | 120 |
|  | 85 | 115 | 95 |
|  | 92 | 88 | 78 |
|  | 89 | 69 | 128 |
| Average SD CV(%) | 91 | 107 | 116 |
|  | 2.6 | 27.7 | 23.9 |
|  | 2.9 | 25.8 | 20.6 |

Table 2

| | Example 1 | Comparative Example 1 | Comparative Example 2 |
|---|---|---|---|
| Measured value (ng/dL) | 908 | 907 | 1069 |
| | 911 | 868 | 1001 |
| | 911 | 933 | 976 |
| | 915 | 927 | 941 |
| | 912 | 919 | 1078 |
| | 905 | 888 | 1067 |
| | 869 | 925 | 910 |
| | 901 | 921 | 1000 |
| | 917 | 917 | 853 |
| | 896 | 914 | 969 |
| Average SD CV(%) | 905 | 913 | 986 |
| | 14.0 | 20.3 | 73.2 |
| | 1.5 | 2.2 | 7.4 |

Table 3

| | Example 1 | Comparative Example 1 | Comparative Example 2 |
|---|---|---|---|
| Measured value (ng/dL) | 2119 | 2201 | 1952 |
| | 2134 | 2111 | 2007 |
| | 2131 | 2217 | 1906 |
| | 2152 | 2110 | 2267 |
| | 2197 | 2081 | 1878 |
| | 2133 | 2189 | 1883 |
| | 2149 | 2177 | 2209 |
| | 2143 | 2150 | 2277 |
| | 2171 | 2176 | 2199 |
| | 2155 | 2237 | 1858 |
| Average SD CV(%) | 2148 | 2165 | 2044 |
| | 22.5 | 50.8 | 173.9 |
| | 1.0 | 2.3 | 8.5 |

(4) Confirmation of dilution linearity in the method for an immunological analysis of the present invention

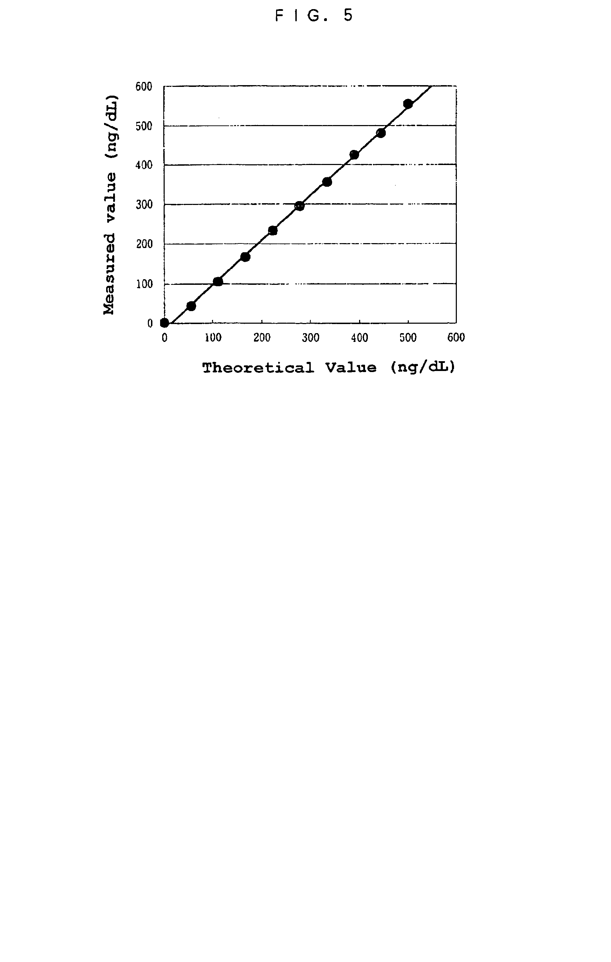

A human serum containing an elastase 1 antigen at a concentration of 6000 ng/dL was used as a standard solution of an elastase 1 antigen. Diluted solutions were prepared by diluting the standard solution to various concentrations listed in Table 4, and then a concentration of elastase 1 in each diluted solution was measured three times per each diluted solution. The measurement was carried out in accordance with the procedure described in Evaluation (1), except that 5 µL of each diluted solution was used instead of 5 µL of the human serum.
The results are shown in Table 4. Correlations between measured values and theoretical values are shown in Figs. 4 and 5. Fig. 4 shows the results in the case that the theoretical values were 0 to 6000 ng/dL, and Fig. 5 shows the results in the case that the theoretical values were 0 to 600 ng/dL. From the results in Fig. 4, Equation (2):

$$y=1.0217x-19.734 \tag{2}$$

was obtained as a correlation equation, and the $R^2$ value was 0.9997. From the results in Fig. 5, Equation (3):

$$y=1.1229x-15.015 \tag{3}$$

was obtained as a correlation equation, and the $R^2$ value was 0.9988. As apparent from Figs. 4 and 5, the method for an immunological analysis of the present invention showed a good dilution linearity.

As apparent from the above evaluations (1) to (4), the method for an immunological analysis of the present invention shows a close correlation with RIA, a wider measuring range than that of the RIA method, and a good reproducibility.

Table 4

| Theoretical value (TV) | Measured value (MV) | MV/TV |
|---|---|---|
| (ng/dL) | (ng/dL) | (%) |
| 0 | 1 | |
| 56 | 43 | 77.9 |
| 111 | 104 | 93.8 |
| 167 | 167 | 99.7 |
| 222 | 233 | 104.6 |
| 278 | 295 | 106.0 |
| 334 | 354 | 106.1 |
| 389 | 425 | 109.2 |
| 445 | 480 | 107.9 |
| 500 | 555 | 110.9 |
| 556 | 613 | 110.2 |
| 1112 | 1186 | 106.6 |
| 1668 | 1745 | 104.6 |
| 2224 | 2355 | 105.9 |
| 2781 | 2882 | 103.7 |
| 3337 | 3415 | 102.4 |
| 3893 | 3962 | 101.8 |
| 4449 | 4559 | 102.5 |
| 5005 | 5190 | 103.7 |
| 5561 | 5637 | 101.4 |
| 7000 | 6860 | 98.0 |
| 8000 | 6981 | 87.3 |
| 9000 | 7475 | 83.1 |
| 10000 | 7798 | 78.0 |
| 14000 | 8329 | 59.5 |

(5) Confirmation of reactivity of the reagent for an immunological analysis of the present invention

In this evaluation example, reactivities of the reagent for an immunological analysis of the present invention prepared in Example 1, with respect to elastase 1 in various forms, were evaluated. The results are shown in Table 5. The results in Table 5 are shown as relative values when the reactivity to a complex of elastase 1 and $\alpha_1$-antitrypsin is regarded as 100%. The abbreviation "PMSF" in Table 5 means phenyl-methanesulfonyl fluoride. As shown in Table 5, the reagent for an immunological analysis of the present invention agglutinated a complex of elastase 1 and $\alpha_1$-antitrypsin, but did not agglutinate a separate (i.e., free) elastase 1.

Table 5

| Substance to be evaluated | Reactivity |
|---|---|
| elastase 1-$\alpha_1$-antitrypsin complex | 100% |
| elastase 1 | 0.9% |
| PMSF-elastase 1 | <0.1% |
| $\alpha_2$ macroglobulin-elastase 1 complex | <0.1% |
| $\alpha_1$-antitrypsin | <0.001% |
| $\alpha_2$ macroglobulin | <0.001% |

Comparative Example 2: Measurement of elastase 1 concentration by EIA method

In this comparative example, a concentration of elastase 1 in each sample used in the above evaluation (2) was measured by an EIA method. The EIA method was carried out as follows. To each well, in which an $F(ab')_2$ fragment of an anti-elastase 1 antibody E1 was immobilized, of a 96-well plastic plate, 10 µL of each sample was added and reacted at 37°C for 2 hours. After washing the wells with a phosphate buffer, a peroxidase-labeled $F(ab')_2$ fragment of an anti-elastase 1 antibody E4 was added to the wells, and reacted at 37°C for an hour. After washing the wells with the phosphate buffer, a hydrogen peroxide solution of o-phenylenediamine was added to the wells, and reacted for 15 minutes. The reaction was stopped by adding a sulfuric acid solution, and then an absorbance at a wave length of 492 nm of each well was measured.

INDUSTRIAL APPLICABILITY

According to the reagent or method for an immunological analysis of the present invention, elastase 1 can be analyzed conveniently without a specific equipment and for a short time (for example, for 10 minutes). Further, according to the reagent or method for an immunological analysis of the present invention, elastase 1 can be analyzed quantitatively in a wide range from a low concentration range to a high concentration range. According to the method for detecting a pancreatic disease, a pancreatic disease (particularly acute pancreatitis) can be detected conveniently and quickly, and thus the detecting method can react to an urgent test.

As above, the present invention is explained with reference to particular embodiments, but modifications and improvements obvious to those skilled in the art are included in the scope of the present invention.

**Claims**

1. A reagent for an immunological analysis of elastase 1, comprising two kinds of latex particles having different particle sizes wherein said latex particles carry two kinds of monoclonal antibodies having different specificities for elastase 1.

2. A reagent for an immunological analysis of elastase 1, comprising first latex particles carrying a first monoclonal antibody specific to elastase 1, and second latex particles having a particle size different from that of the first latex particle and carrying a second monoclonal antibody having a specificity different from that of the first monoclonal antibody.

3. A method for an immunological analysis of elastase 1, comprising the steps of:

   bringing a sample to be analyzed into contact with two kinds of latex particles having different particle sizes, said latex particles carrying two kinds of monoclonal antibodies having different specificities for elastase 1, and analyzing the degree of a latex-particles-agglutination caused by an antigen-antibody reaction.

4. A method for an immunological analysis of elastase 1, comprising the steps of:

   bringing a sample to be analyzed into contact with first latex particles carrying a first monoclonal antibody specific to elastase 1, and second latex particles having a particle size different from that of the first latex particle and carrying a second monoclonal antibody having a specificity different from that of the first monoclonal antibody, and

analyzing the degree of a latex particles agglutination caused by an antigen-antibody reaction.

5.   The method for an immunological analysis according to claim 3 or 4, wherein the latex particles are brought into contact with the sample to be analyzed at a pH range of 6 to 8.5.

6.   A method for detecting a pancreatic disease wherein elastase 1 is analyzed by the method for an immunological analysis according to claim 3 or 4.

7.   A method for detecting a pancreatic disease wherein elastase 1 is analyzed by the method for an immunological analysis according to claim 5.

F I G. 1

F I G. 2

F I G. 3

F I G. 4

F I G. 5

## INTERNATIONAL SEARCH REPORT

| International application No. |
| --- |
| PCT/JP02/03255 |

| A. CLASSIFICATION OF SUBJECT MATTER |
| --- |
| Int.Cl⁷  G01N33/543, G01N33/577, G01N33/573, G01N33/545 |

According to International Patent Classification (IPC) or to both national classification and IPC

| B. FIELDS SEARCHED |
| --- |

Minimum documentation searched (classification system followed by classification symbols)
Int.Cl⁷  G01N33/50-33/98

Documentation searched other than minimum documentation to the extent that such documents are included in the fields searched
Jitsuyo Shinan Koho       1922-1996   Toroku Jitsuyo Shinan Koho   1994-2002
Kokai Jitsuyo Shinan Koho  1971-2002   Jitsuyo Shinan Toroku Koho   1996-2002

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | JP 10-123137 A  (Sekisui Chemical Co., Ltd.), 15 May, 1998 (15.05.98), (Family: none) | 1-7 |
| Y | JP 63-73152 A  (Dainabot Co., Ltd.), 02 April, 1988 (02.04.88), (Family: none) | 1-7 |
| Y | JP 03-215747 A  (Maruko Seiyaku Kabushiki Kaisha), 20 September, 1991 (20.09.91), (Family: none) | 1-7 |
| Y | JP 02-304365 A  (Shionogi & Co., Ltd.), 18 December, 1990 (18.12.90), & EP 398621 A          & DE 69015253 C & CA 2014703 A1    .      & AT 116063 T | 1-7 |

☐ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 11 June, 2002 (11.06.02) | 25 June, 2002 (25.06.02) |

| Name and mailing address of the ISA/ Japanese Patent Office | Authorized officer |
| --- | --- |
| Facsimile No. | Telephone No. |

Form PCT/ISA/210 (second sheet) (July 1998)